**Europäisches Patentamt**

**European Patent Office**

**Office européen des brevets**

(19)

(11) Veröffentlichungsnummer : **0 361 139 B1**

# EUROPÄISCHE PATENTSCHRIFT

(12)

(45) Veröffentlichungstag der Patentschrift :
**15.01.92 Patentblatt 92/03**

(51) Int. Cl.$^5$ : **A61M 5/00**

(21) Anmeldenummer : **89116339.6**

(22) Anmeldetag : **05.09.89**

(54) **Spritzentablett.**

(30) Priorität : **26.09.88 DE 8812156 U**
**13.07.89 DE 3923119**
**23.08.89 DE 8910067 U**

(43) Veröffentlichungstag der Anmeldung :
**04.04.90 Patentblatt 90/14**

(45) Bekanntmachung des Hinweises auf die
Patenterteilung :
**15.01.92 Patentblatt 92/03**

(84) Benannte Vertragsstaaten :
**AT BE CH DE FR IT LI NL**

(56) Entgegenhaltungen :
**EP-A- 0 192 453**
**DE-U- 8 711 864**
**DE-U- 8 901 550**
**US-A- 4 420 085**
**US-A- 4 485 918**

(73) Patentinhaber : **HAMMERLIT GMBH**
**Sägemühlenstrasse 49**
**W-2950 Leer/Ostfriesland (DE)**

(72) Erfinder : **Meseke, Curt Th.**
**Gartenstrasse 16**
**W-2950 Leer/Ostfriesland (DE)**
Erfinder : **Winkler, Winfried**
**Schwinningstrasse 20**
**W-5100 Aachen (DE)**
Erfinder : **Hilbig, Renate**
**Am Obernhof 21**
**W-4700 Hamm 1 (DE)**

(74) Vertreter : **Rehders, Jochen, Dipl.-Ing.**
**Stresemannstrasse 28**
**W-4000 Düsseldorf 1 (DE)**

EP 0 361 139 B1

## Beschreibung

Die Erfindung betrifft ein Spritzentablett zum Lagern und Transportieren von medizinischen Spritzen mit einer Kanüle und einer Kanülenkappe, mit einem Tablettboden.

Durch das DE-GM 8815519 ist eine Vorrichtung zur Erleichterung der Verabreichung von medizinischen Spritzen in Form eines Tabletts bekannt, in dessen Tablettfläche Aufnahmefächer für Spritzen, Ampullen, Kanülen, Ampullensägen usw. eingeformt sind. Mit diesem Behälter kann ein Behälter für gebrauchte Kanülen auswechselbar verbunden werden. — Durch das DE-GM 8801602 ist ein Tablett für medizinische Einsätze mit ggf. unterteiltem Boden und Längs- und Querrandpartien bekannt, das eine ringsumlaufende im allgemeinen rechteckförmig konstruierte Einfassung bildet. Hierbei ist die Tablettfläche durch Zwischenwände als Erhebungen unterteilt, in denen Vertiefungen vorgesehen sind, die zum Lagern von Spritzen dienen.

Derartige Tabletts wie auch der Erfindungsgegenstand dienen dem Transport von medizinischen Hilfsmitteln so auch von Injektions- und Blutentnahmespritzen. — Für eine Injektion oder eine Blutentnahme wird die auf der Kanülennadel gehaltene Kanülenkappe entfernt und hierdurch die Nadel zum Einstich freigegeben. Die freiliegende und nach dem Einstich kontamenierte Kanülennadel stellt nach der Injektion oder der Blutentnahme ein Infektionsrisiko für den Arzt insbesondere im Hinblick auf eine Hepatitis- oder AIDS-Infektion dar, wenn das Einführen der Kanülennadel in die Kanülenkappe in der Weise erfolgt, daß mit der einen Hand die Spritze geführt und mit der anderen Hand die Kanülenkappe gehalten wird.

Durch die US-PS 4596562 ist ein Handgerät bekannt, mit dem dieses Risiko gemindert werden soll und das mit Bohrungen unterschiedlichen Durchmessers versehen ist, in die Kanülenkappen eingesteckt werden können. Beim Einführen der Kanülennadel in die Kanülenkappe muß hierbei das Gerät mit der einen Hand gehalten werden, während mit der anderen die Spritze gehalten und deren Kanülennadel in die Kappe eingeführt wird. Gegenüber dem Festhalten der Kanülenkappe wird der Vorteil erzielt, daß das Gerät im Abstand von der Kanüle von der einen Hand gehalten wird, gleichwohl aber diese Haltehand des Benutzers noch im Bereich der Kanülennadel liegt. Abgesehen hiervon bedarf es jeweils der beiden Hände des Arztes um die Kanülennadel in die Kanülenkappe einzuführen. — Bohrungen in diesem Gerät sind derart ausgebildet, daß die Kanülenkappe klemmend in dem Gerät gehalten wird, so daß es zum Lösen der auf die Kanülennadel gesteckten Kanülenkappe des Herausdrückens der Kanülenkappe aus der Bohrung durch Beaufschlagung des Kanülenbodens bedarf. — Ein ähnliches Gerät zeigen die EP-OS 0192453 und die US-PS 4485918. In der EP-A-0192453 wird die Kanülenkappe von einer Bohrung klemmend gehalten, wobei die seitlichen Flansche des Geräts den Daumen und den Zeigefinger der Haltehand des Benutzers schützen sollen. Wird das Gerät mit der eingeführten Kappe auf eine Unterlage, z.B. eine Tischplatte gelegt, so muß die Kappe in dem Gerät festgehalten werden, was durch das Klemmen in der Bohrung oder dem hülsenartigen Abschnitt des Gerätes erfolgt, um ein Einführen der benutzten Nadel in die Kappe zu ermöglichen.

Beachtenswert ist somit bei diesem Gerät der Grundgedanke des Klemmens der Kanülenkappe in dem Gerät. Hierbei muß darauf geachtet werden, daß beim Lösen der Kappe aus dem Handgerät die Verbindung zwischen dem Kopfteil der Kanülennadel und dem entsprechenden Ende der Kappe, die klemmend zusammengehalten werden, nicht gelöst wird, da andernfalls die Aufgabe der Kappe als Nadelschutz verlorengeht. Die Kappe muß daher bei dem beschriebenen bekannten Gerät herausgedrückt werden.

Der Erfindung liegt die Aufgabe zugrunde, ein Spritzentablett zu schaffen, bei welchem jegliches Berühren und damit Verletzen Benutzungsperson der kontaminierten Kanülennadel ausgeschlossen ist, das Einstecken der Kanülennadel in die Kanülenkappe ohne Zuhilfenahme der zweiten Hand erfolgen kann und ferner das Herausnehmen der Kanülenkappe aus dem Tablett ein Erfassen oder Beaufschlagen des Bodens der Kanülenkappe überflüssig macht. Dies bedeutet, daß das Tablett auf seiner Unterlage belassen werden kann, um die Kanülenkappe mit der Nadel klemmend zu verbinden. Dies soll bei einfacher Gestaltung des Tabletts möglich sein.

Zur Lösung dieser Aufgabe sind zwei Ausführungsformen der Erfindung vorgesehen, die in den Ansprüchen 1 und 2 ihren Niederschlag finden. Die erste Ausführungsform geht von einem Wulst aus, in dem die Aufnahmebohrungen für die Kanülenkappen vorgesehen sind. Die zweite Ausführungsform sieht zylinderförmige Erhebungen vor, in die die Kanülenkappe einsteckbar ist. — Die Merkmale der Unteransprüche dienen der Weiterbildung und Verbesserung des Haupt- und des Nebenanspruches.

Der Vorteil des erfindungsgemäßen Spritzentabletts ist darin zu sehen, daß es nur des Lösens der Kanülenkappe von der Nadel und des Einführens der Kappe stehend in die Bohrung oder den Zylinder bedarf. Die Kanülenkappe befindet sich hierbei in senkrechter oder annähernd senkrechter Lage und stützt sich auf der Unterlage ab, auf der das Tablett aufsteht. Da ein Spritzentablett auf einer Unterlage in einer Bodenhöhe von 0,60-1,20 m aufsitzt, kann ein sicheres Einführen der gebrauchten Nadel in die Kappe von oben durch den Benutzer erfolgen. Da die Kappe ungeklemmt in der Bohrung und dem Zylinder steht, wird die Kappe nicht in der Bohrung oder dem Zylinder zurückgehalten, wenn die Spritze mit der gebrauchten Nadel und der Kappe

EP 0 361 139 B1

nach oben von dem Tablett entfernt und in eines der Tablettabteile abgelegt wird.

Auf der Zeichnung sind Ausführungsbeispiele des erfindungsgemäßen Tabletts dargestellt und zwar zeigt

| | |
|---|---|
| Fig. 1 und 2 | eine perspektivische Ansicht einer Ausführungsform des Tabletts nach der Erfindung und einen Schnitt nach der Linie II-II der Fig. 1, |
| Fig. 3 und 4 | eine weitere Ausführungsform des erfindungsgemäßen Spritzentabletts mit mehreren in Reihe gelegenen zylindrischen Erhebungen und einen Schnitt nach der Linie IV-IV der Fig. 3, |
| Fig. 5 | eine Abwandlung der Ausführungsform der Fig. 4, |
| Fig. 6 | ein weiteres Ausführungsbeispiel eines Spritzentabletts mit einem mittleren Wulst und darin angeordneten Bohrungen unterschiedlichen Durchmessers, |
| Fig. 7, 8 und 9 | einen in einem Wulst nach Fig. 1 und 2 gehaltenen Einsatz, |
| Fig. 10 | die Ausführung des Tablettrandes einer weiteren Ausführungsmöglichkeit des erfindungsgemäßen Tabletts, |
| Fig. 11 und 12 | eine weitere Ausführungsform in perspektivischer Ansicht und einen Schnitt nach der Linie XI-XI der Fig. 11, |
| Fig. 13 | Die Lage und Stellung der Teile bei der Ausführungsform nach Fig. 11. |

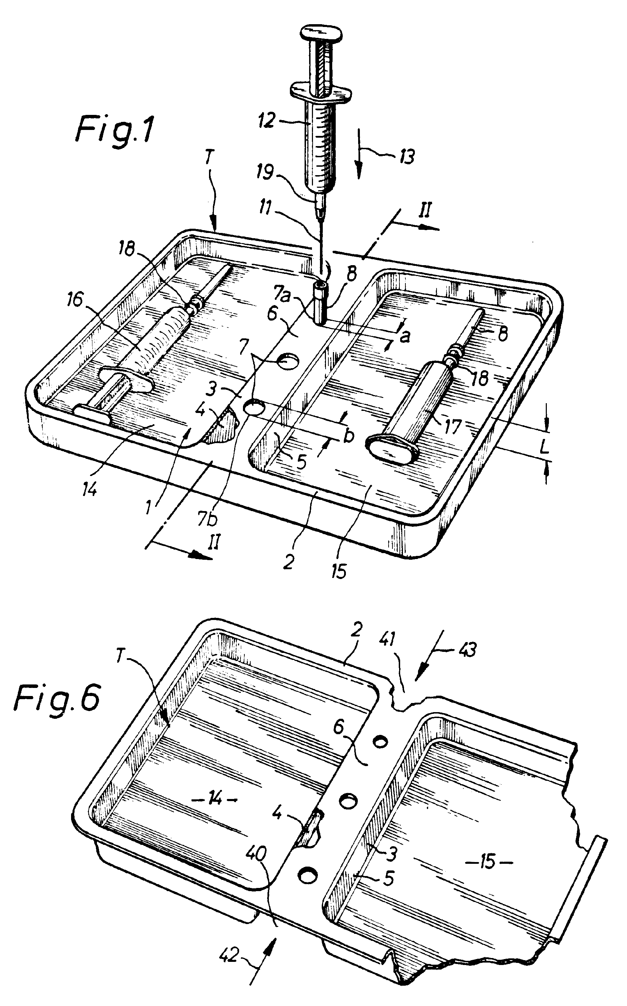

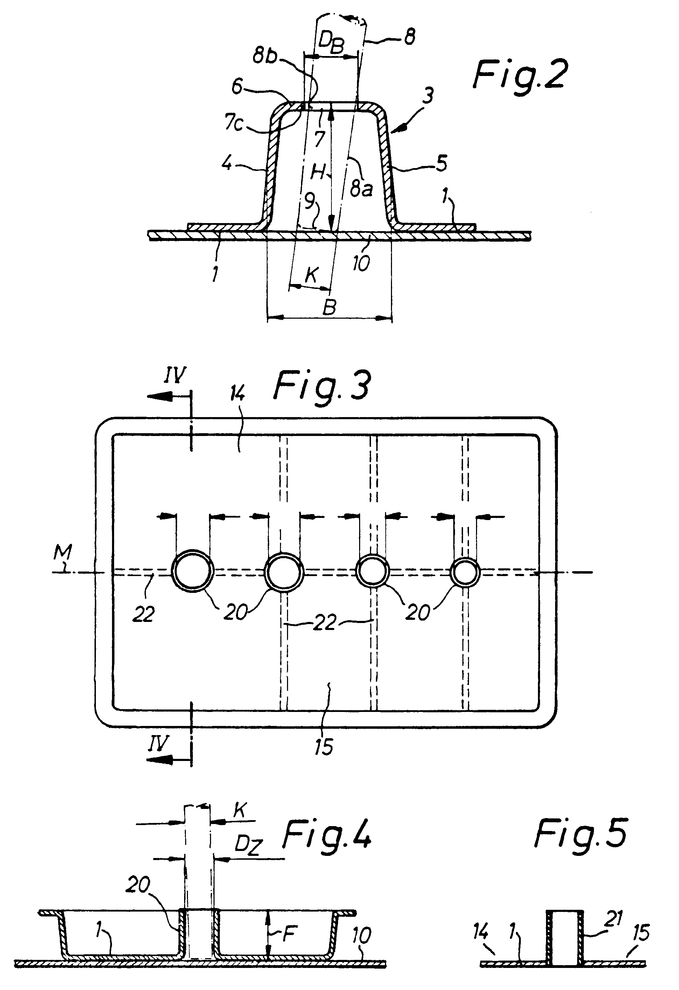

Das Tablett T nach der Erfindung weist einen auf einer nicht dargestellten Unterlage aufsitzenden Boden 1 auf, der von einem umlaufenden hochgebogenen kragenartigen Rand 2 begrenzt wird. Aus der Ebene des Bodens 1 erhebt sich bei der Ausführungsform nach Fig. 1 in der Längs- oder Quermitte des Tabletts ein Wulst 3, der die beiden Seitenwände 4, 5 sowie die Wulstoberwand 6 besitzt. Die Höhe H (Fig. 2) des Wulstes 3 kann der Höhe L des Randes 2 entsprechen, wobei der Wulst vorzugsweise eine mittlere Breite B besitzt, die größer als die Breite des Randes 2 ist.

Das Tablett T besteht aus gespritztem Kunststoffmaterial mit für seinen besonderen Einsatzzweck entsprechenden Eigenschaften. Die Oberwand 6 des vorzugsweise im Querschnitt trapez- oder rechteckförmigen Wulstes 3 weist eine Reihe von im Abstand voneinander angeordnete Bohrungen 7 auf, die unterschiedliche Durchmesser aufweisen und zwar entsprechend dem Durchmesser handelsüblicher Kanülenkappen. So z.B. kann die Bohrung 7a einen Durchmesser a und die Bohrung 7b einen größeren Durchmesser b aufweisen. Der Durchmesser $D_B$ der Bohrungen 7 ist gleich oder kleiner als die Höhe H des Wulstes 3, so daß die in die Bohrung 7 eingesetzte Kanülenkappe 8 senkrecht oder annähernd senkrecht steht, wenn sie mit ihrem Boden 9 auf der Unterlage 10 des Tablettes T z.B. einem Tisch oder dem Regal eines Instrumentenwagens aufsitzt.

Die Relation zwischen der Öffnung $D_B$ und dem Durchmesser K der Kanülenkappe 8 soll so gewählt werden, daß die Kanülenkappe möglichst keine geringere Neigung als 60° aufweist, wenn sie durch die Bohrung 7 mit ihrem Fußabschnitt 8a eingesetzt ist. Vorzugsweise sollte die Kappe 8 eine senkrechte Stellung, wie sie in Fig. 1 gezeigt ist, einnehmen, so daß die gebrauchte Kanülennadel 11 der Spritze 12 in senkrechter Richtung (vgl. Pfeil 13 in Fig. 1) in die Kanülenkappe 8 von oben eingeführt werden kann. Hierdurch ist ein Höchstmaß an Treffsicherheit der Kanülennadel 11 in die Kappe 8 gewährleistet.

Die verschiedenen Durchmesser der Bohrungen 7 gewährleisten, daß ein senkrechtes Stehen der Kanülenkappe 8 in den Bohrungen 7 gegeben ist. Selbst dann wenn der Benutzer sich in der Wahl des Durchmessers der Bohrung 7 geirrt hat, bleibt dennoch die Gewähr, daß ein weitgehend senkrechtes Stehen der Kanülenkappe in der Bohrung 7 gegeben ist.

Die Kanülenkappe 8 ist in der Bohrung 7 derart gehalten, daß noch ein Spiel zwischen dem Rand 8b der Kanülenkappe und dem Innenrand 7c der Bohrung 7 gegeben ist. Dies bedeutet, daß die Kanülenkappe 8 frei in der Bohrung 7 steht und in dieser nicht geklemmt wird.

Der Wulst 3 unterteilt das Tablett in zwei Abteilungen 14, 15 derart, daß in dem Abteil 14 die nicht benutzten Spritzen 16 mit den Kanülennadeln und Kappen Aufnahme finden können, während die benutzten Spritzen 17 mit den entsprechenden Kanülennadeln und den Kappen in dem Abteil 15 abgelegt werden können.

Der Benutzer einer Spritze entnimmt dem Abteil 14 die unbenutzte Spritze und entfernt die Kanülenkappe 8, die in die entsprechende Bohrung 7 des Wulstes 3 in die Lage nach Fig. 2 gebracht wird. Nach Verabreichung der Injektion oder der Blutentnahme wird dann, wie Fig. 1 erkennen läßt, die Kanülennadel 11 in die stehende Kanülenkappe 8 eingeführt, deren Kopf 18 klemmend mit dem entsprechenden konisch ausgebildeten Kopf 19 der Kanülennadel 11 verbunden wird. Hieraufhin kann die Spritze 12 mit der gebrauchten Kanülennadel 11 und der aufgeklemmten Kanülenkappe 8 entgegengesetzt zur Pfeilrichtung 13 der Bohrung 7 und dem Wulst 3 entnommen werden. Hiernach kann die Spritze mit der kontaminierten Nadel 11 und der Kappe 8 in das Abteil 15 gelegt werden.

Bei der Ausführungsform nach Fig. 3 ist statt eines Wulstes 3 der Ausführungsform nach Fig. 1 und 2 eine Reihe von Zylindern 20 wiederum in der Längs- oder Quermitte M des Tabletts T vorgesehen. Dieses besitzt

3

EP 0 361 139 B1

wiederum einen ringsumlaufenden Rand 2, wobei die Länge F der Zylinder gleich oder größer als der Durchmesser $D_Z$ und der Zylinder 20 soviel größer als der mittlere Durchmesser K der Kappe 8 ist, daß mit freiem Bewegungsspiel des Fusses 8a der Kappen 8 in den Zylindern 20 steht.

Die Zylinder 20 können auch hier eine Länge F besitzen, die der Höhe L des Randes 2 entspricht.

In Fig. 5 ist eine andersartige, mögliche Bildung des Zylinders 20 nach Fig. 3 und 4 dargestellt.

Auch bei dieser Ausführungsform wird durch die Reihe der Zylinder 20 eine Unterteilungswand gebildet, durch die das Tablett in die Abteile 14 und 15 gemäß Fig. 1 unterteilt wird. Zusätzliche flossenartige Wände 22 können eine weitere Unterteilung des Tabletts bringen.

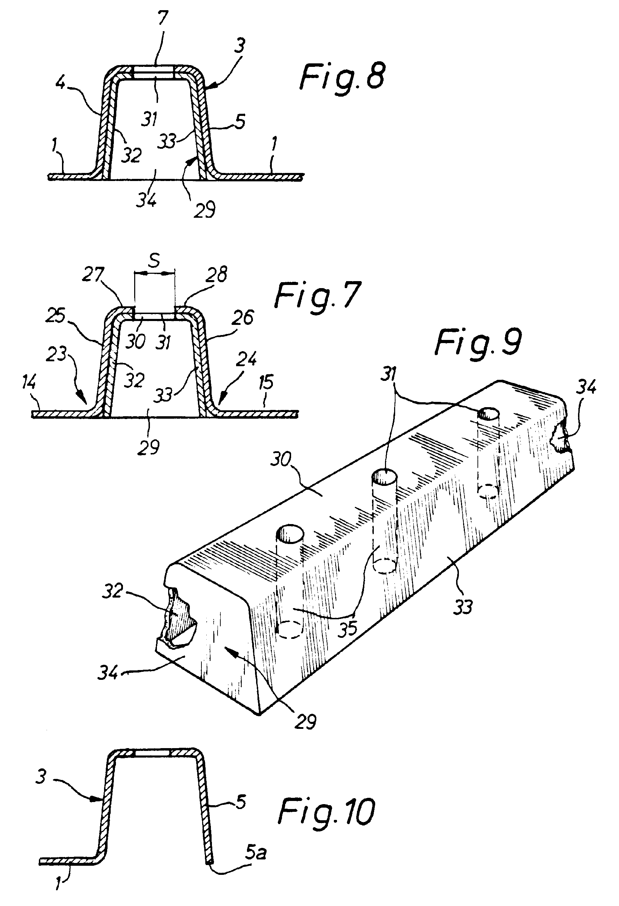

Bei der Ausführungsform nach Fig. 7 besteht das Tablett T aus zwei Einzelteilen 23, 24 mit Seitenwänden 25, 26. Diese gehen in ihrem oberen Ende in schmale horizontale leistenförmige Abschnitte 27, 28 über, die einen Abstand S voneinander haben. Bei dieser Ausführungsforn findet ein im Querschnitt trapezförmiger Einsatzblock 29 (Fig. 9) Anwendung, dessen Oberwand 30 wiederum in Reihe und im Abstand voneinander angeordnete Bohrungen 31, vorzugsweise unterschiedlichen Durchmessers aufweisen. Ein solcher Einsatzblock wird mit seinen Seitenwänden 32, 33 mit den Wänden 25, 26 der beiden Einzeltabletteile verklebt, so daß wiederum ein integrales Tablett mit den Abteilen 14, 15 entsteht. Auf diese Art und Weise lassen sich Tabletts aus mehreren Einzelteilen, wie die Teile 23, 24, zusammensetzen, um Tabletts verschiedener Größe in Baukastenweise zu gewinnen. Hierbei sind die Abschnitte 27, 28 der Tabletteinzelteile wie umlaufende kragenartige Ränder ausgebildet, die den Rändern 2 nach Fig. 1, 2 entsprechen können. Das Einsatzteil kann zur Erhöhung der Stabilität des Tabletts mit Kopfwänden 34 versehen oder massiv sein.

Um ein quer- oder längssteifes Tablett zu erhalten, kann der blockförmige Einsatz 30 nach Fig. 9 auch bei einer Ausführungsform eines Tabletts nach Fig. 1 und 2 Anwendung finden. Hierbei werden die Seitenwände 32, 33 des blockförmigen Einsatzes 30 mit den Wulstseitenwänden 4, 5 verschweißt oder verklebt (Fig. 8).

Der Einsatz 30 nach Fig. 9 kann auch mit rohrförmigen Teilen 35 versehen werden, die den Zylindern 20 nach Fig. 3-5 entsprechen.

Letztendlich ist in Fig. 10 dargestellt, daß der Wulst 3 auch als Rand eines Tabletts gestaltet sein kann, wobei der untere Rand 5a der Seitenwand 5 des Wulstes 3 auf Höhe des Bodens 1 des Tabletts enden kann.

Die Anordnung des Wulstes 3 und dessen Bohrungen 7 sowie der Zylinder 10 ist so gewählt, daß keine "toten" Ecken oder Bereiche entstehen und von daher das Tablett leicht zu reinigen und zu desinfizieren ist ; der Wulst 3 wie auch die Zylinder 20 sind hierzu nach unten offen. Diesem Bestreben entspricht auch die Ausführungsforn nach Fig. 6, bei der die Enden 40, 41 des Wulstes 3 offen sind, wobei die Wulstwände 4, 5 und die Deckwand 6 der Steifigkeit des Tablettes entsprechend ausgebildet sind. Bei dieser Ausführungsform kann ein Reinigen der Innenseite des Wulstes 3 aus der Richtung der Pfeile 42, 43 vorgenommen werden.

Um ohne Schwierigkeiten und Zeitverlust diejenige Bohrung sicher zu erkennen, deren Durchmesser den Durchmesser der jeweils benutzten Kappe 8 entspricht, um dieser eine möglichst senkrechte Stellung in dem Wulst zu geben, kann dieser von seinem anderen Ende eine deutlich sichtbare Verbreiterung erfahren, die der Zunahme des Durchmessers der in Reihe gelegenen Bohrungen entspricht. Bei einer Vergrößerung des Durchmessers a der Bohrung 7a in Fig 1 zum Durchmesser b der übernächsten Bohrung verbreitert sich der Wulst 3 entsprechend. — Auch kann die Länge der Zylinder 20 dem ansteigenden Durchmesser gemäß Fig. 3 zunehmen. Auch kann die Höhe des Wulstes vergleichsweise mit wachsendem Bohrungsdurchmesser sich vergrößern.

Der Wulst 3 wie die Reihe der Zylinder 20 kann auch eine andersartige als die dargestellte Orientierung aufweisen ; so kann der Wulst bzw. die Zylinderreihe schräg und über eine Ecke des Tabletts verlaufen.

Die Abmessungen der Wulsthöhe, des Bohrungsdurchmessers bzw. die Länge der Zylinder 20 und deren Durchmesser werden, wie vorstehend dargelegt, so gewählt und aufeinander abgestimmt, daß die Kappe 8 senkrecht oder weitgehend senkrecht steht, wobei ein Klemmen der Kappe in der Bohrung bzw. den Zylindern mit einer Kraft unterbleibt, durch die beim gemeinsamen Herausziehen der Kanüle und der Kappe aus der Bohrung bzw. dem Zylinder die Klemmverbindung zwischen dem Nadelkopf 18 und der Kanüle 8 aufgehoben und die Nadel unbeabsichtigt von der Kappe entfernt wird. Auf jeden Fall muß beim Abheben oder Entfernen des Spritzenkörpers mit der Nadel die Kappe mitgenommen werden, ohne daß die Kappe von der Tablettunterseite einen Längsschub erfährt.

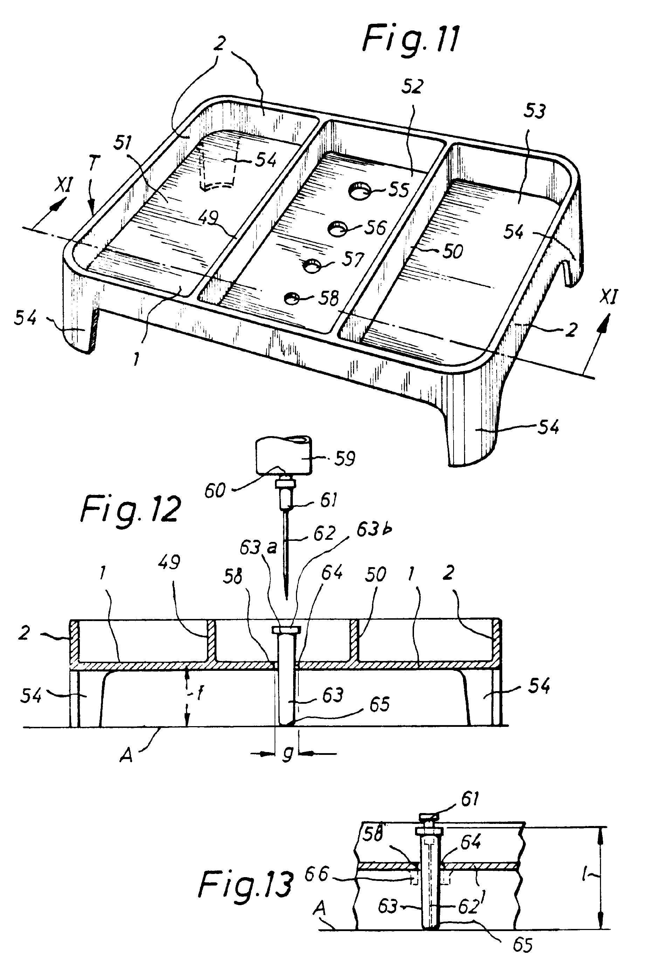

Bei der Ausführungsform nach Fig. 11 bis 13 weist das Tablett T wiederum einen Tablettboden 1 auf, der eben ist und parallel zu einer Tablettauflage A verläuft. Der Tablettboden 1 wird umschlossen von einem ringsumlaufenden kragenartigen Rand 2 ; ferner befinden sich im mittleren Abschnitt des Tablettes Trennwände 49, 50, durch die zusammen mit dem Rand 2 Abteilungen 51, 52, 53 gebildet werden, von denen mindestens die Abteilungen 51 und 53 zur Aufnahme von medizinischen Spritzen dienen. — Die Größe des Tablettes ist für diesen Zweck ebenso gewählt wie die Größe der Abteilungen 51 und 53. Das Tablett besitzt an seinen vier Ecken Füße 54, durch die der Boden 1 einen Abstand F von der Tablettauflage A erhält. Dieser Abstand hat eine bestimmte Größe, die nachstehend noch erläutert wird.

4

Die mittlere Abteilung 52 des Tabletts weist eine Reihe von Bohrungen 55, 56, 57, 58 auf, die unterschiedlichen Durchmesser besitzen, wobei der Durchmesser von der größten Bohrung 55 zur kleinsten Bohrung 58 kontinuierlich abnimmt. — Die Bohrungen oder ihre Umgebung können mit Farbmarkierungen versehen sein, durch die das Erkennen des Bohrungsdurchmessers erleichtert wird.

Auch hier weisen die medizinischen Spritzen einen Spritzenkörper 59 mit einem hohlen konischen Ansatz 60 auf. Auf diesen ist der hohle gleichfalls konische Kopf 61 der Kanülennadel aufsteckbar und wird dort klemmend gehalten. Der Kanülennadel 62 ist eine Kappe 63 zugeordnet, die auch hier den Zweck hat, die Kanülennadel 62 aufzunehmen, und die mit ihrem oberen Abschnitt 63a klemmend auf dem konischen Kopf 61 der Kanülennadel 62 gehalten wird. Der Größe der Injektionsspritze entsprechend besitzen die Kanülenkappen 63 unterschiedliche Durchmesser, denen die unterschiedlichen Durchmesser der Bohrungen 55-58 Rechnung tragen.

Die Bohrungen 55-58 haben einen Durchmesser g, der zu den Kanülenkappen verschiedener Größen stets einen kleinen Zwischenraum 64 beläßt, der ein freies Bewegungsspiel der Kanülenkappe 63 in den Bohrungen 55-58 ermöglicht, wobei, wie in Fig. 12 und 13 dargestellt ist, die Kanülenkappe 63 eine stehende oder weitgehend stehende Stellung einnimmt und hierbei der Fuß 65 auf der Auflage A aufsitzt. Dieses nicht klemmende Stehen der Kanülenkappe 63 in den Bohrungen 55-58 ist insofern wichtig, als beim Einstecken der Kanülennadel 62 in die Kappe 63 eine klemmende Verbindung zwischen dem Kopf 61 der Kanülennadel 62 und dem Ende 63a der Kappe 63 einerseits eintritt und beim Aufwärtsbewegen des Spritzenkörpers 59 die Kappe 63 andererseits aus der Bohrung 55-58 frei herausgezogen werden kann, ohne daß die Kappe 63 in den Bohrungen 55-58 festgehalten wird und es der Zuhilfenahme der zweiten Hand der Benutzungsperson des Tabletts bedarf, um die Kappe von der Unterseite des Tabletts her aus der Bohrung herauszustoßen. Hierdurch ist jede Gefahr des Einstichs der kontaminierten Kanülennadel 62 in die zweite Hand des Benutzers ausgeschlossen. — Mit anderen Worten : auch hier muß die Klemmkraft zwischen dem Kopf 61 und dem oberen konischen Abschnitt 63a der Kappe 63 garantiert größer sein als die Reibung der Kappe an der Bohrungswand. — Im wesentlichen klemmfrei in diesem Sinne bedeutet daher, die geschilderte Verbindung der Kappe mit dem Nadelkopf einerseits und das Herausziehen der mit dem Kopf 61 verbundenen Kappe aus der Bohrung andererseits.

Die Höhe F des Bodens 1 über der Auflage A ist so gewählt, daß die Kappe 63 eine stehende oder weitgehend stehende im wesentlichen klemmfreie Lage einnimmt, wie dies vorstehend beschrieben ist. Vorzugsweise soll hierbei der Abstand F nicht kleiner als 1/5 der Länge I der längsten Kappe sein.

Um die stehende oder weitgehend stehende Stellung der Kanülenkappen 63 zu unterstützen, können die Ränder der Bohrungen 55-58 nach unten oder oben gegen die Auflage A kragenartig umgebörtelt sein, wie dies mit dem Kragen 66 in Fig. 13 erkennbar ist.

Aus Vorstehendem geht hervor, daß die Kappe 63 mit ihrem Fuß 65 auf der Tablettauflage A aufsteht und hierbei in den Bohrungen 55-58 nicht oder nicht nennenswert klemmend gehalten wird. — Die Kappe 63 kann jedoch auch in den Bohrungen hängen ; dann ist der Abstand F zwischen der Aufstellfläche A und dem Boden 1 größer als der Abstand zwischen der Aufstellfläche A und der ringartigen Erweiterung oder Wulstes 63b (Fig. 12). Hier hängt dann die Kappe 63 mit ihrer Erweiterung oder ihrer Wulst auf dem Rand der Bohrungen aufliegend im wesentlichen klemmfrei in den Bohrungen 55-58.

## Patentansprüche

1. Spritzentablett zum Lagern und Transportieren von medizinischen Spritzen mit einer Kanüle und einer Kanülenkappe, mit einem Tablettboden (1) und mit einer aus der Bodenebene nach oben herausragenden Erhebung **dadurch gekennzeichnet, daß** die Erhebung ein Wulst (3) mit einer oder mehreren Bohrungen (7) in seiner oberen Wulstwand (6) zum Einführen von Kanülenkappen (8) ist, wobei die Kappen (8) im wesentlichen klemmfrei in dem Wulst (3) stehend anzuordnen sind, oder daß die Erhebung von einem oder mehreren, nach oben offenen Zylindern (20) gebildet ist, wobei die Kappen im wesentlichen klemmfrei in den Zylindern (20) stehend anzuordnen sind (Fig. 1-10).

2. Spritzentablett zum Lagern und Transportieren von medizinischen Spritzen mit einer Kanüle und einer Kanülenkappe, mit einem Tablettboden **dadurch gekennzeichnet, daß** der Tablettboden (1) mit einer oder mehreren Bohrungen (55-58) zum im wesentlichen klemmfreien Einführen der Kanülenkappen (63) versehen und in einem Abstand von 1/5 der Länge bis zu mehr als der Länge der Kappe bzw. der längsten Kappe (63) von der Aufstellfläche (A) des Tabletts (T) anzuordnen ist (Fig. 11-13).

3. Spritzentablett nach Anspruch 1, **dadurch gekennzeichnet, daß** der Durchmesser ($D_B$, $D_Z$) der Wulstbohrung (7) bzw. des Zylinders (20) um mindestens ein Bewegungsspiel der Kanülenkappe (8) in der Bohrung (7) bzw. dem Zylinder (20) gröber als der mittlere Durchmesser (K) der Kanülenkappe ist.

4. Spritzentablett nach Anspruch 1, dadurch gekennzeichnet, daß der Zylinder (20) an seinem unteren Ende offen ist.

5. Spritzentablett nach Anspruch 1, dadurch gekennzeichnet, daß der Wulst (3) die Tablettfläche in Fächer oder Abteile (14, 15) unterteilt.

6. Spritzentablett nach Anspruch 1, dadurch gekennzeichnet, daß mehrere Zylinder (20) in einer Reihe angeordnet sind, die die Tablettfläche in Fächer oder Abteile (14, 15) unterteilt.

7. Spritzentablett nach Anspruch 1 mit einem Tablettrand, dadurch gekennzeichnet, daß der Wulst (3) bzw. der Zylinder (20) eine der Höhe des kragenartigen Tablettrandes (2) entsprechende Höhe bzw. Länge aufweist.

8. Spritzentablett nach Anspruch 1, dadurch gekennzeichnet, daß die Zylinder (20) in einem in den Wulst (3) eingesetzten Block (29) angeordnet sind.

9. Spritzentablett nach Anspruch 1, dadurch gekennzeichnet, daß der Zylinder (20) als Rohrstück mit seinem Fuß auf den Tablettboden (1) aufgeschweißt oder in den Wulst (3) eingeschweißt ist.

10. Spritzentablett nach Anspruch 1, dadurch gekennzeichnet, daß der Tablettboden (1) als Wanne oder Wannenbereiche aus der Ebene des Wulstes (3) und des kragenartigen Tablettrandes (2) tiefgezogen ist.

11. Spritzentablett nach Anspruch 1 und einem der Ansprüche 2 bis 10, dadurch gekennzeichnet, daß der Wulst (3) sich von einem zum anderen Ende verbreitert.

12. Spritzentablett nach Anspruch 11, dadurch gekennzeichnet, daß die Verbreiterung des Wulstes (3) der Zunahme des Durchmessers (d) der aufeinander folgenden mehreren Bohrungen (7) des Wulstes entspricht.

13. Spritzentablett nach Anspruch 1 und 2, dadurch gekennzeichnet, daß die Höhe des Wulstes (3) bzw. die Länge der Zylinder (20) dem zunehmenden Durchmesser der mehreren Bohrungen (7) oder mehreren Zylinder (20) entsprechend zunimmt.

14. Spritzentablett nach Anspruch 1 und einem oder mehreren der Ansprüche 2 bis 13, dadurch gekennzeichnet, daß die Höhe des Wulstes (3) gleich oder größer als der mittlere Durchmesser ($D_B$) der im Innern des Wulstes (3) stehenden Kanülenkappe (8) ist.

15. Spritzentablett nach Anspruch 1, dadurch gekennzeichnet, daß die Länge des Zylinders (20) gleich oder größer ist als der mittlere Durchmesser ($D_Z$) der in dem Zylinder (20) stehenden Kanülenkappe (8).

16. Spritzentablett nach Anspruch 1 und 2, dadurch gekennzeichnet, daß die Bohrung oder Bohrungen um mindestens ein Bewegungsspiel der Kappe oder Kappen in der Bohrung oder den Bohrungen größer als der Kappendurchmesser ist.

17. Spritzentablett nach Anspruch 1 und 2, dadurch gekennzeichnet, daß die mehreren Bohrungen des Tabletts unterschiedliche Durchmesser haben.

18. Spritzentablett nach Anspruch 1 oder 2 und einem der Ansprüche 3 bis 17, dadurch gekennzeichnet, daß der Rand der Bohrungen (7, 55-58) oder der Zylinder (20) in axialer Richtung der Bohrungen oder Zylinder kragenartig nach oben oder nach unten umgebogen ist.

19. Spritzentablett nach Anspruch 2, dadurch gekennzeichnet, daß der Abstand (F) zwischen der Aufstellfläche (A) und dem Tablettboden (1) größer als der Abstand (1) der ringartigen Erweiterung oder des Wulstes (63b) am Kappenkopf (63b) der Kappe (63) ist, so daß die in eine Bohrung (55-58) einführbare Kappe (63) im wesentlichen klemmfrei in den Bohrungen (55-58) hängend anzuordnen ist.

20. Spritzentablett nach Anspruch 1 oder 2 und einem oder mehreren der Ansprüche 3 bis 19, dadurch gekennzeichnet, daß die Bohrungen (7, 55-58) oder die Zylinder (20) oder deren Umgebung unterschiedlich farbig zum besseren Erkennen der Bohrungs- oder Zylinderdurchmesser gehalten wird.

## Claims

1. Syringe tray for storing and transporting medical syringes with a cannula and a cannula cap, having a tray floor (1) and having an elevation projecting upwards from the floor level, characterized in that the elevation is a beading (3) with one or several boreholes (7) in its upper beading wall (6) for the insertion of cannula caps (8), whereby the caps (8) are to be arranged so that they stand in a manner substantially without clamping in the beading (3), or that the elevation is formed from one or several cylinders (20) which are open to the top, whereby the caps are to be arranged so that they stand in a manner substantially without clamping in the cylinders (20) (Figures 1-10).

2. Syringe tray for storing and transporting medical syringes with a cannula and a cannula cap, having a tray floor, characterized in that the tray floor (1) is provided with one or several boreholes (55-58) for the insertion, substantially without clamping, of the cannula caps (63) and is to be arranged at a distance of 1/5 of the length up to more than the length of the cap or of the longest cap (63) from the mounting surface (A) of the tray (T) (Figures 11-13).

3. Syringe tray according to claim 1, characterized in that the diameter ($D_B$, $D_Z$) of the beading borehole

6

(7) or of the cylinder (20) is greater than the middle diameter (K) of the cannula cap by at least one clearance of motion of the cannula cap (8) in the borehole (7) or the cylinder (20).

4. Syringe tray according to claim 1, characterized in that the cylinder (20) is open at its lower end.

5. Syringe tray according to claim 1, characterized in that the beading (3) subdivides the tray surface into compartments or divisions (14, 15).

6. Syringe tray according to claim 1, characterized in that several cylinders (20) are arranged in a row which subdivides the tray surface into compartments or divisions (14, 15).

7. Syringe tray according to claim 1 with a tray rim, characterized in that the beading (3) or the cylinder (20) has a height or length corresponding to the height of the collar-like tray rim (2).

8. Syringe tray according to claim 1, characterized in that the cylinders (20) are arranged in a block (29) inserted into the beading (3).

9. Syringe tray according to claim 1, characterized in that the cylinder (20) is welded as pipe piece with its base on to the tray floor (1) or is welded into the beading (3).

10. Syringe tray according to claim 1, characterized in that the tray floor (1) is deep-drawn as trough or trough regions from the level of the beading (3) and the collar-like tray rim (2).

11. Syringe tray according to claim 1 and one of claims 2 to 10, characterized in that the beading (3) widens from one end to the other.

12. Syringe tray according to claim 11, characterized in that the widening of the beading (3) corresponds to the increase of the diameter (d) of the several successive boreholes (7) of the beading.

13. Syringe tray according to claim 1 and 2, characterized in that the height of the beading (3) or the length of the cylinders (20) increases corresponding to the increasing diameter of the several boreholes (7) or several cylinders (20).

14. Syringe tray according to claim 1 and one or several of claims 2 to 13, characterized in that the height of the beading (3) is the same as or greater than the middle diameter ($D_B$) of the cannula cap (8) standing in the inside of the beading (3).

15. Syringe tray according to claim 1, characterized in that the length of the cylinder (20) is the same as or greater than the middle diameter ($D_Z$) of the cannula cap (8) standing in the cylinder (20).

16. Syringe tray according to claim 1 and 2, characterized in that the borehole or boreholes is/are larger than the cap diameter by at least one clearance of motion of the cap or caps in the borehole or the boreholes.

17. Syringe tray according to claim 1 and 2, characterized in that the several boreholes of the tray have differing diameters.

18. Syringe tray according to claim 1 or 2 and one of claims 3 to 17, characterized in that the rim of the boreholes (7, 55-58) or of the cylinders (20) in the axial direction of the boreholes or cylinders is bent round upwards or downwards in a collar-like manner.

19. Syringe tray according to claim 2, characterized in that the distance (F) between the mounting surface (A) and the tray floor (1) is greater than the distance (1) of the annular extension or of the beading (63b) at the cap head (63b) of the cap (63), so that the cap (63) which can be inserted into a borehole (55-58) is to be arranged so that it hangs in a manner substantially without clamping in the boreholes (55-58).

20. Syringe tray according to claim 1 or 2 and one or several of claims 3 to 19, characterized in that the boreholes (7, 55-58) or the cylinders (20) or their surroundings carry different colours for better recognition of the borehole or cylinder diameters.

**Revendications**

1. Tablette à seringues, pour stocker et transporter des seringues médicales comportant une canule et un capuchon de canule, avec un fond (1) et un bossage, faisant saillie vers le haut en partant du plan de fond, caractérisée en ce que le bossage est un bourrelet (3) doté d'un ou plusieurs perçages (7) dans sa paroi supérieure (6), pour introduire des capuchons de seringue (8), les capuchons (8) devant être disposés debout dans le bourrelet (7) pratiquement sans aucun serrage, ou en ce que le bossage est formé par un ou plusieurs cylindres (20), ouverts vers le haut, les capuchons devant être disposés debout dans les cylindres (20), pratiquement sans aucun serrage (figures 1 à 10).

2. Tablette à seringues, pour stocker et transporter des seringues médicales comportant une canule et un capuchon de canule, avec un fond de tablette, caractérisée en ce que le fond (1) est pourvu d'un ou plusieurs perçages (55 à 58), en vue d'introduire, pratiquement sans aucun serrage, les capuchons de canules (63) et qu'il est à disposer à une distance de la face de pose (A) de la tablette (T) qui va de 1/5 de la longueur jusqu'à plus de la longueur du capuchon, ou du capuchon le plus long (63) (figures 11 à 13).

3. Tablette à seringues selon la revendication 1, caractérisée en ce que le diamètre ($D_B$, $D_Z$) du perçage de bourrelet (7), ou du cylindre (20), est supérieur, d'au moins la valeur d'un ajustement avec jeu de déplacement du capuchon de canule (8) dans le perçage (7), ou dans le cylindre (20), au diamètre moyen (K) du capuchon de canule.

4. Tablette à seringues selon la revendication 1, caractérisée en ce que le cylindre (20) est ouvert à son extrémité inférieure.

5. Tablette à seringues selon la revendication 1, caractérisée en ce que le bourrelet (3) subdivise la surface de tablette en casiers ou compartiments (14, 15).

6. Tablette à seringues selon la revendication 1, caractérisée en ce que plusieurs cylindres (20) sont disposés en une rangée qui subdivise la surface de tablette en casiers ou compartiments (14,15).

7. Tablette à seringues selon la revendication 1, avec une bordure de tablette, caractérisée en ce que le bourrelet (3), ou le cylindre (20), présente une hauteur, ou une longueur, correspondant à la hauteur de la bordure de tablette (2) en collerette.

8. Tablette à seringues selon la revendication 1, caractérisée en ce que les cylindres (20) sont disposés dans un bloc (29) inséré dans le bourrelet (3).

9. Tablette à seringues selon la revendication 1, caractérisée en ce que le cylindre (20) est un tube soudé, avec son pied, sur le fond de tablette (1), ou soudé au sein du bourrelet (3).

10. Tablette à seringues selon la revendication 1, caractérisée en ce que le fond (1), faisant office de cuvette ou de zones de cuvette, est formé par étirage profond, depuis le plan du bourrelet (3) et de la bordure de tablette (2) en collerette.

11. Tablette à seringues selon la revendication 1, caractérisée en ce que le bourrelet (3) s'élargit en allant d'une extrémité à l'autre.

12. Tablette à seringues selon la revendication 1, caractérisée en ce que l'élargissement du bourrelet (3) correspond à l'accroissement du diamètre (d) des différents perçages (7) du bourrelet qui se suivent.

13. Tablette à seringues selon les revendications 1 et 2, caractérisée en ce que la hauteur du bourrelet (3), ou la longueur des cylindres (20) augmente de manière correspondant au diamètre croissant des différents perçages (7) ou des différents cylindres (20).

14. Tablette à seringues selon la revendication 1 et une ou plusieurs des revendications 2 à 13, caractérisée en ce que la hauteur du bourrelet (3) est égale ou supérieure au diamètre moyen ($D_B$) du capuchon de canule (8) placé debout à l'intérieur du bourrelet (3).

15. Tablette à seringues selon la revendication 1, caractérisée en ce que la longueur du cylindre (20) est égale ou supérieur au diamètre moyen ($D_Z$) du capuchon de canule (8) placé debout dans le cylindre (20).

16. Tablette à seringues selon les revendications 1 et 2, caractérisée en ce que le ou les perçages sont plus grands que le diamètre de capuchon, de la valeur d'au moins un ajustement à jeu de déplacement du ou des capuchons dans le ou les perçages.

17. Tablette à seringues selon les revendications 1 et 2, caractérisée en ce que les différents perçages de la tablette ont des diamètres différents.

18. Tablette à seringues selon les revendications 1 et 2 et l'une des revendications 3 à 17, caractérisée en ce que le bord des perçages (7, 55 à 58) ou des cylindres (20) sont incurvés vers le haut ou vers le bas, dans la direction axiale des perçages ou cylindres, à la façon de collerettes.

19. Tablette à seringues selon la revendication 2, caractérisée en ce que la distance (F) entre la surface de pose (A) et le fond de tablette (1) est supérieure à la distance (1) entre l'élargissement annulaire ou du bourrelet (63b) situé sur la tête de capuchon (63b) du capuchon (63), de sorte que le capuchon (63) pouvant être introduit dans un alésage (55 à 58) doive être disposé pratiquement sans aucun serrage et suspendu dans les perçages (55-58).

20. Tablette à seringues selon les revendications 1 et 2 et l'une des revendications 3 à 19, caractérisée en ce que les perçages (7, 55 à 58) ou les cylindres (20), ou leur entourage sont colorés différemment, pour améliorer l'identification des diamètres de perçages ou de cylindres.

*Fig.1*

*Fig.6*

Fig.2

Fig.3

Fig.4

Fig.5

*Fig.8*

*Fig.7*

*Fig.9*

*Fig.10*

*Fig.11*

*Fig.12*

*Fig.13*